# EUROPEAN PATENT SPECIFICATION

(11) **EP 2 732 780 B1**
(45) Date of publication and mention of the grant of the patent: **11.01.2017**
(21) Application number: 12193116.6
(22) Date of filing: 16.11.2012
(51) Int. Cl.: A61B 17/3213, A61B 17/3211

(54) **Retractable universal safety scalpel**
Einziehbares universelles Sicherheitsskalpell
Scalpel de sécurité universel rétractable

(43) Date of publication of application: 21.05.2014
(73) Proprietor: Paramount Surgimed Ltd., 110 020 New Delhi (IN)
(72) Inventor: Kumar, Abhay, New Delhi 110 020 (IN)
(74) Representative: Moore, Michael Richard

(56) References cited:
- CN-U- 202 143 644
- US-A- 5 299 357
- US-A- 5 599 351
- US-A- 5 626 596
- US-A1- 2007 078 472
- US-B1- 6 254 621

## Description

### FIELD OF THE INVENTION

The present invention relates to a surgical tool and more particularly to a retractable universal safety scalpel having safety features and means movable along the scalpel handle to move the blade mounted in the said handle between a pre-use un-exposed position to in-use exposed position and to an after-use unexposed position thereby permanently covering and locking the blade.

### BACKGROUND OF THE INVENTION

Surgical cutting instruments such as scalpels are widely used to perform surgery. The disposable surgical scalpels are well known in the art and often comprise a handle, typically formed of a plastic material, to which is attached either permanently or detachably, a scalpel blade. In operation, the handle is dealt with to get the scalpel blade exposed for use. This, of course, also exposes the blade to all individuals, doctors, nurses, medical technicians, etc., associated with a surgical procedure, as well as those individuals who are involved with the disposal of the used scalpel. Even with the exercise of great care, individuals are frequently inadvertently cut by the exposed scalpel blade. The dangers of being cut and transmission of infectious diseases when cut by a used scalpel blade are thus ever-present. Thus, the individuals handling the surgical scalpel remain at risk.

Further, the potential for blood born infection has created a demand for surgical instruments which in protective position shields permanently the blade once used thereby providing protection to the user from inadvertent dangers of being cut. A wide variety of scalpels with retractable blades are available in the industry. Nevertheless, there remains a continuing need to improve the performance of such devices so that they meet the users' expectations in terms of performance as a surgical instrument as well as offer improved safety.

Moreover, there remains a constant need for a surgical scalpel which provides improved control and safety features reducing the amount of pressure required to be exerted by the surgeon to maintain control of the scalpel thereby making the handling and operation of the scalpel much easier.

US 5,599,351 A, which forms the basis of the preamble of claim 1, describes scalpel constructions that enable a scalpel to have its cutting edge retracted to a shielded 'sharp-safe' position and locked in that shielded position to avoid after-use infection of others, especially by diseases transmitted in blood.

### SUMMARY OF THE INVENTION

The disclosed surgical tool relates to a retractable universal safety scalpel comprising a handle having a proximal section and a distal section with a longitudinally extending bore defining a housing; a blade carrier element having mid-section, proximal end and a distal end on which is secured a blade slidably mounted within said housing being capable of getting exposed from the distal end section of said housing; first and second slilder elements secured to said blade carrier element through a recess formed in the handle extending from a forward end closer to an opening to a rear end closer to the distal section of the handle in a slidable arrangement with the housing and extending externally in an axial direction of said housing to move said blade carrier element moving the blade from an un-exposed pre-use position to an exposed in-use position and to an after-use position capable of covering and locking permanently the blade completely within the housing of the handle; a plurality of locking means is provided in the housing to enable locking of the blade in an un-exposed pre-use position to an exposed in-use position and to an after-use position capable of covering and locking permanently the blade, wherein the housing is provided with lock cooperating mechanisms at three positions thereon corresponding to pre-use, in-use and post-use disposal stages, said lock cooperating mechanisms cooperating with the respective locking means provided thereon. The invention is characterised in that the first and second slider elements comprise on their inner surfaces a forward tab positioned near the distal end thereof and a rear tab positioned near the proximal end thereof; the blade carrier element is provided with first and second flexible latching elements extending generally in angular opposing directions adjoining the mid-section of the blade carrier element, such that when secured in the housing the latching elements slidably engage with the first and second slider elements; and the housing comprises a pair of locking members for engagably receiving the slider elements and retaining them securely in the permanently locked after-use position.

Further features of the invention provides for a first and second slider element movably mounted in the said handle for sliding movement of the said blade carrier element comprising the blade relative to said handle between a pre-use unexposed position to in-use exposed position and to an after-use unexposed position allowing the said blade to move to each of said positions when the afore-said slider elements are activated.

Further features of the invention provide for the said first and second slider elements to be in a movably locking / engaging arrangement with the recess provided in the axial direction opening through and between opposite sides of the handle, said sliding members being provided with one or more engagement means, for example tooth and notch formations that cooperate to hold the said blade carrier element and slider members in one or more locking and / or engagement arrangements capable of being movable in each of the said pre-use, in-use and after-use positions such that a person operating the scalpel will know exactly, by feel and be aware of the position and movement of the blade member in the said housing of the said handle. It will also be appreciated that the engagement means being in engaging arrangement with said recess is shaped for cooperation with the margins of the said recess to maintain the slider members in positions being smoothly movable in each of the afore-said positions.

Further features of the invention provides for a retractable universal safety scalpel wherein said blade carrier element member includes a blade. Guide surfaces are provided in the inner surface of the housing of the said handle in an engaging arrangement with said blade carrier and movable along said handle in response to sliding movement of said slider elements in each of the said pre-use, in-use and after-use positions.

Further features of the invention provide for the said blade carrier of the said retractable universal safety scalpel being configured such that it can accept blades having plurality of different profiles and/or sizes thereby making it universal in use.

Further features of the invention provides for a retractable universal safety scalpel, which in use position allows for direct finger control of the scalpel handle and blade without interfering or getting interfered from the said slider elements. Moreover, the slider elements are slidable along the handle between all positions being adapted to move the slider elements between its pre-use position and in-use position. Further, the slider elements are slidable between pre-use position and in-use positions multiple times, whereby the scalpel may be used, set aside and then reused with the movable slider members.

Further features of the invention provides for a retractable universal safety scalpel wherein said slider elements are movable during the course of in-use position allowing the user to have the exposure of desired length of the surgical blade.

Further features of the invention provides for a retractable universal safety scalpel wherein said slider elements are movable into a permanent lock position covering / locking permanently the said blade in the housing of the said handle.

Further features of the invention provides for locking means in the housing to enable locking of the blade in an un-exposed pre-use position to an exposed in-use position and to an after-use position capable of covering and locking permanently the said blade member wherein the said housing being provided with lock cooperating mechanisms at three positions thereon corresponding to pre-use, in-use and post-use disposal stages, said lock cooperating mechanisms cooperating with the respective locking means provided thereon.

Further features of the invention provides for a retractable universal safety scalpel which can be easily used / handled by both a right-handed as well as left-handed person.

Further features of the invention provides for a retractable universal safety scalpel including one or more safety elements having safety features such as locking safety features to prevent completely the accidental retraction of the blade when in use and/or providing permanent lock for the blade after use.

Methods of use and construction of the retractable universal safety scalpel are also described.

### BRIEF DESCRIPTION OF THE DRAWINGS

The present invention will now be explained in more detail in the following with reference to the preferred embodiments and to the accompanying drawings, wherein:
FIGS. 1 and 2 illustrate right and left side exploded perspective views of the retractable universal safety scalpel, where the assembly elements are detached from the scalpel cover according to the present invention;
FIGS. 3 and 4 illustrate right and left side exploded perspective views of the retractable universal safety scalpel, where the blade carrier element is slidably attached in the housing of the scalpel cover exposing the blade in-use position according to the present invention;
FIGS. 5 and 6 illustrate right and left side exploded perspective views of the retractable universal safety scalpel, where the first and second slider elements are movably mounted respectively in the recess of the scalpel cover exposing the blade in-use position according to the present invention;
FIGS. 7 and 8 illustrate right and left side views of the assembled retractable universal safety scalpel exposing the blade in-use position according to the present invention.
FIG. 9 illustrates the top view of the assembled retractable universal safety scalpel exposing the blade in-use position according to the present invention.
FIGS. 10 and 11 illustrate right and left side see-through views of the retractable universal safety scalpel where the blade carrier element is slidably engaged with the distal end of the scalpel handle exposing the blade in-use position according to the present invention.
FIGS. 12 and 13 illustrate right and left side views of the blade carrier element according to the present invention.
FIGS. 14 and 15 illustrate perspective views from the right and left side of the blade carrier element according to the present invention.
FIGS. 16A - 16D collectively referred to as FIG. 16 illustrate right, left, front and perspective views of the first slider element in the open position according to the present invention.
FIGS. 17A -17D collectively referred to as FIG. 17 illustrate right, left, front and perspective views of the second slider element in the open position according to the present invention.
FIGS. 18A - 18B collectively referred to as FIG. 18 illustrate perspective views from the right and left side and top view of the handle according to the present invention
FIGS. 19 to 21 illustrate another embodiment of the retractable universal safety scalpel including locking members to lock the blade carrier element with the blade in a locking position.
FIGS. 22 to 24 illustrate another embodiment of the slider elements of the retractable universal safety scalpel.
FIGS. 25 and 26 illustrate another embodiment of the retractable universal safety scalpel exposing the blade in-use position.

### DETAILED DESCRIPTION OF THE INVENTION

Generally speaking, the term "proximal" refers to a region of the device or parts thereof or a location on the device which is closest to, for example, a user using the device. In contrast to this, the term "distal" refers to a region of the device which is farthest from the user, for example, the distal region of a surgical scalpel will be the region forming the opening from which the blade mounted in the housing of the handle gets exposed when the scalpel is in-use position.

The term "permanently lock position" as used herein means covering the blade member of the surgical scalpel in such a manner that it cannot be un-covered or exposed without destroying the handle of the surgical scalpel.

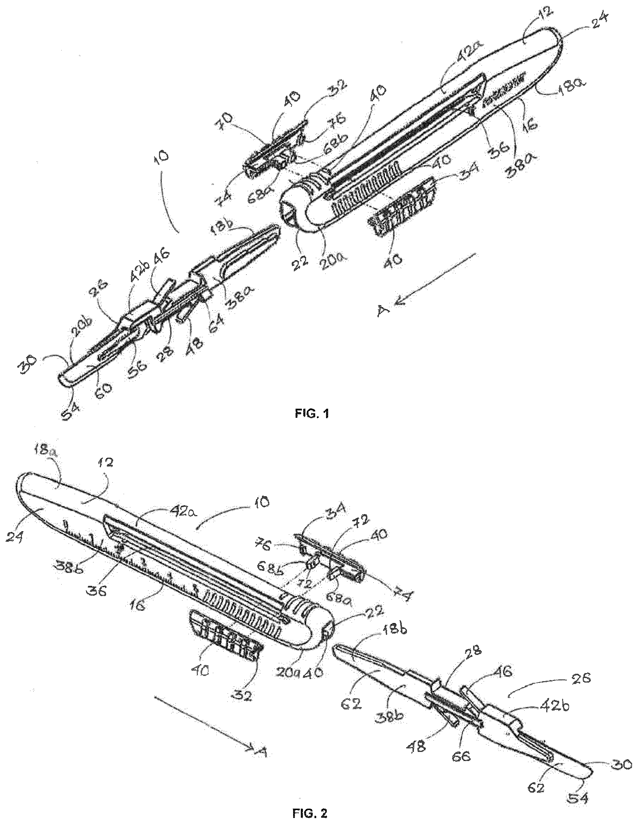

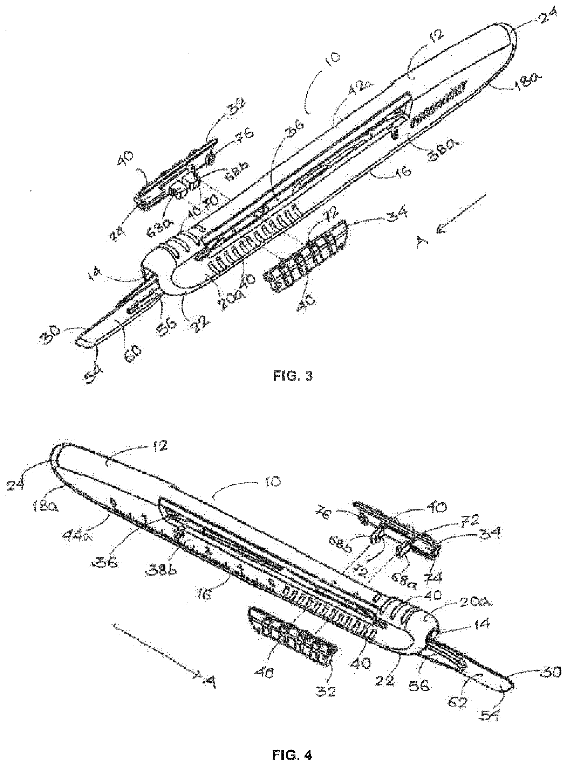

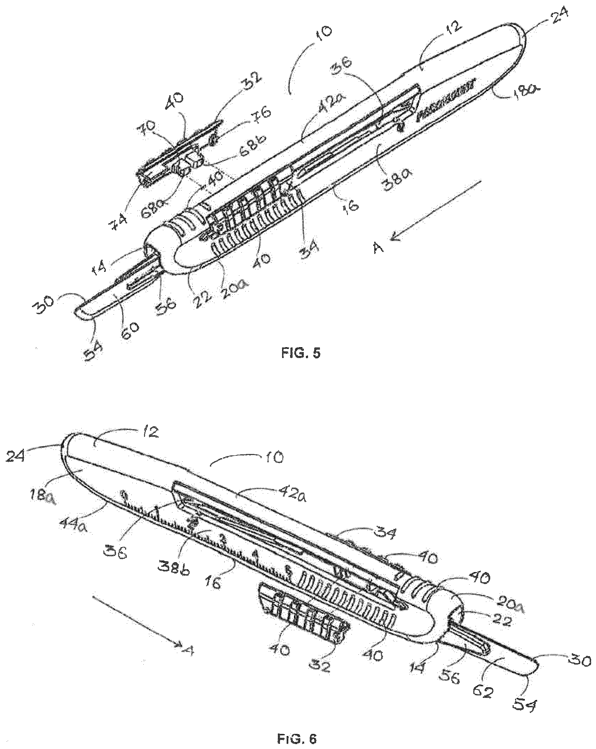

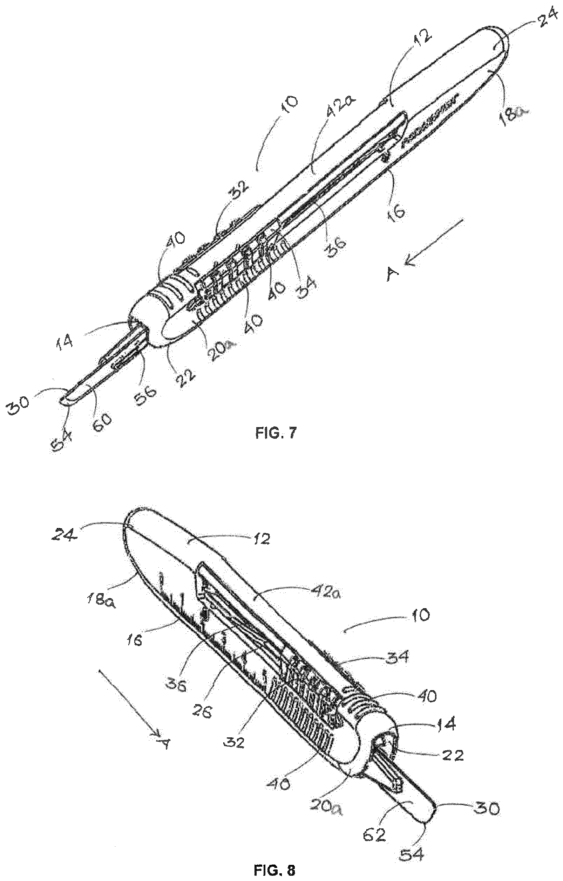

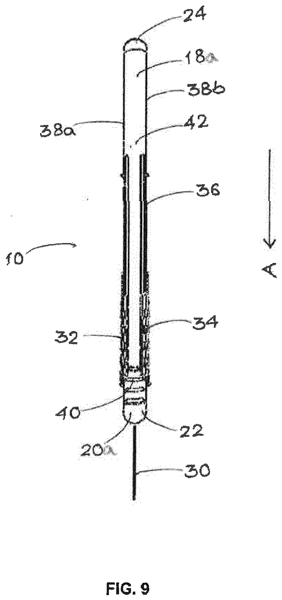

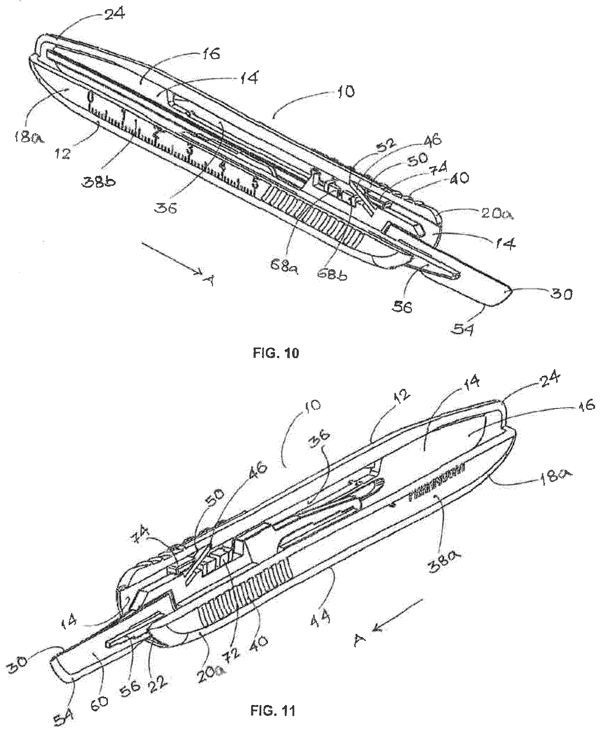

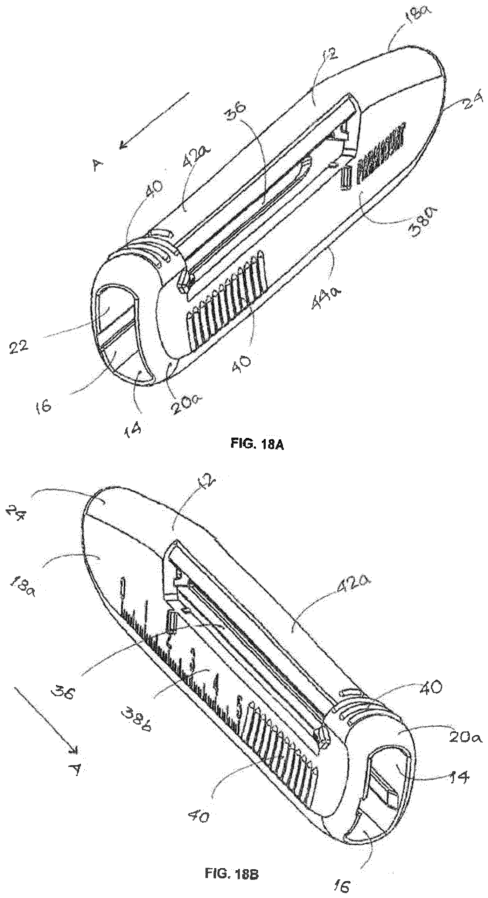

Referring now in detail to the FIGS. 1 to 18 wherein like reference numerals represent like parts throughout the views illustrated, the retractable universal safety scalpel 10 comprises a handle 12 having a proximal 18a section and a distal 20a section with a longitudinally extending bore 14 defining a housing 16, the housing 16 having a opening 22 at the distal 20a end and a closed end 24 at the proximal 18a section of the said handle 12, a blade carrier element 26 having mid-section 28, proximal 18b end and a distal 20b end on which is secured a blade 30 slidably mounted within said housing 16 being capable of getting exposed from the said opening 22 at the distal 20a section of said housing 16; first and second slilder elements secured to said blade carrier element 26 through a recess 36 formed in the said handle 12 extending from the forward end closer to the said opening 22 to the rear end closer to the distal 20a section of the said handle 12 in a slidable arrangement with the said housing 16 and extending externally in an axial direction - A of said housing 16 to move said blade carrier 26 moving the said blade 30 from an un-exposed pre-use position to an exposed in-use position and to an after-use position capable of covering and locking permanently the said blade 30 completely within the said housing 16 of the said handle 12.

As can be seen FIGS. 1 to 11 and 18 the handle 12 defining a housing 16 comprises a generally rectangular shape with substantially rounded outer edges. The distal 20a section of the handle 12 is configured as an opening 22 and the proximal 18a section forms the rear end of the handle 12. The handle 12 is provided with a bore 14 extending in an axial direction - A from opening 22 at the distal 20a end towards the rear end at the proximal 18a section. The side walls of the handle 12 are provided with a recess 36 from the forward end closer to the opening 22 to the rear end closer to the distal 20a section of the handle 12. The recess 36 is designed as a cut out. It extends over the substantial part of handle 12 region, and stretches over the greater part of the blade carrier element 26 comprising the blade 30. As can be seen in FIGS. 1 to 11, the recess 36 is designed such that a substantially equal wall strength results in the entire safety scalpel 10, which is especially advantageous in the manufacture of the retractable universal safety scalpel 10 by an injection molding process for example out of plastic or other suitable matter. One skilled in the art will understand that the recesses can also be designed having other shapes and in particular smaller, for instance as circular hollows or other geometric forms.

The handle 12 also comprises finger grip formation on the top 42a and side wall surfaces 38a, 38b of the handle 12 at the distal 20a section thereof. The grip formation includes one or more ribs 40 forming frictional contact with an individual's fingers. The invention embodies alternate positions of one or more similar ribs 40 along the outer surface of the handle 12. The ribs 40 facilitate gripping the scalpel 10 by the surgeon during use.

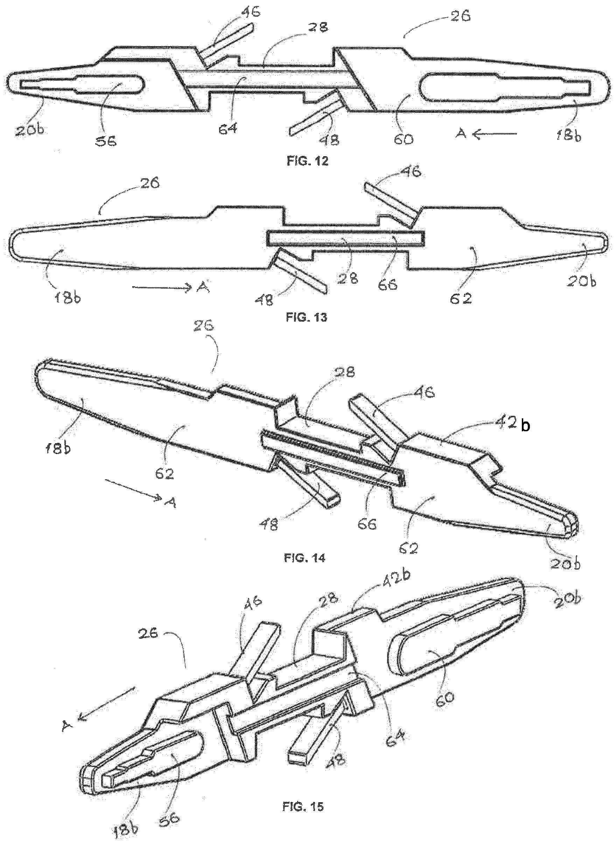

As shown in FIGS. 12 to 15, the blade carrier element 26 comprises several important aspects of the invention in that the distal 20b end forming the forward part and the proximal 18b end forming the rear part is widened compared with mid section 28 so that top 42b and bottom 44b surfaces of the forward and rear part slidably engage the top and bottom surfaces of the bore 14 within the housing 16. The blade carrier element 26 combine with first 32 and second 34 slider elements being connected to the recess 36 provide substantially all the support between the cover housing 16 and the blade 30 secured to the blade carrier element 26. The blade carrier element 26 is also provided with first 46 and second 48 flexible latching elements extending in angular opposing direction adjoining the mid section 28 of the blade carrier element 26. The first 46 and second 48 flexible elements slidably engage with the first 32 and second 34 slider elements when secured in the housing 16. Further, the distal 20a section of the recess 36 has a increased cross-sectional area 50 having dimension of abutment 52 such that the first latching element abuts against the abutment when the blade 30 is in its forward open position being exposed out of the said opening 22.

Further, the blade carrier element 26 includes a blade 30. The blade 30 may be integrally formed with the blade carrier element 26 or may be detachable member capable of being secured with the said blade carrier element 26. The blade 30 includes a cutting edge 54 at the distal 20c end thereof and designed to mate with the mating protrusion 56 positioned near the distal 20b end of the blade carrier element 26. As such, the blade 30 defines an elongated aperture such as a slot 58 near the distal 20c end which in the mating position with the blade carrier element 26 is received onto the mating protrusion 56 positioned near the distal 20b end at the front face of the blade carrier element 26. The mating protrusion 56 formed onto the blade carrier element 26 is thus adapted to securely engage the aperture of the blade 30. During assembly of the blade carrier 26, the blade 30 is fixed to the blade carrier 26 by aligning the aperture of the blade 30 and mating protrusion 56 of the blade carrier element 26. Once the blade 30 aperture is received onto the mating protrusion 56 of the blade carrier 26; it prevents completely the blade 30 from disengaging with the blade carrier element 26. Alternatively, the blade 30 can be connected to the blade carrier element 26 through insert moulding, such that the blade carrier element 26 is formed around the blade 30 during the manufacturing process. Further, the blade 30 has a similar front and back face. The blade 30 can be made of a variety of suitable materials including, but not limited to, carbon and/or stainless material.

The blade carrier element 26 is configured to slide longitudinally within the bore 14 and to receive and support a variety of different profiles of scalpel blades 30. The blade carrier element 26 is elongate in shape and has front and back face having a mid section 28 and proximal 18b and distal 20b ends. The front and back face of the blade carrier element 26 are dissimilar, wherein the mid section 28 of the blade carrier element 26 has a longitudinally extending substantially bar shaped groove 64 on the front face. As opposed to this, the mid section 28 of the blade carrier element 26 on the back face is provided with a longitudinally extending substantially bar shaped projection 66. The inner surface of the handle 12 defining housing 16 is designed to receive the said groove 64 and projection 66 configured on the front and back faces of the blade carrier element 26 in cooperating projection 66 and groove 64 respectively from the inner side wall of the handle 12.

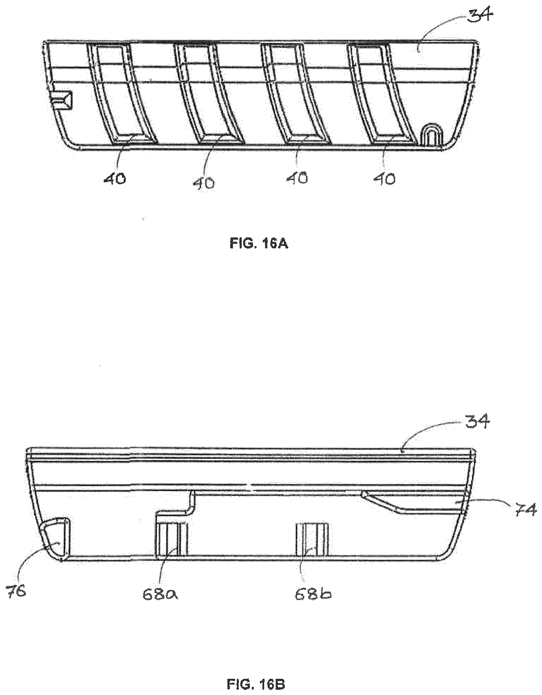

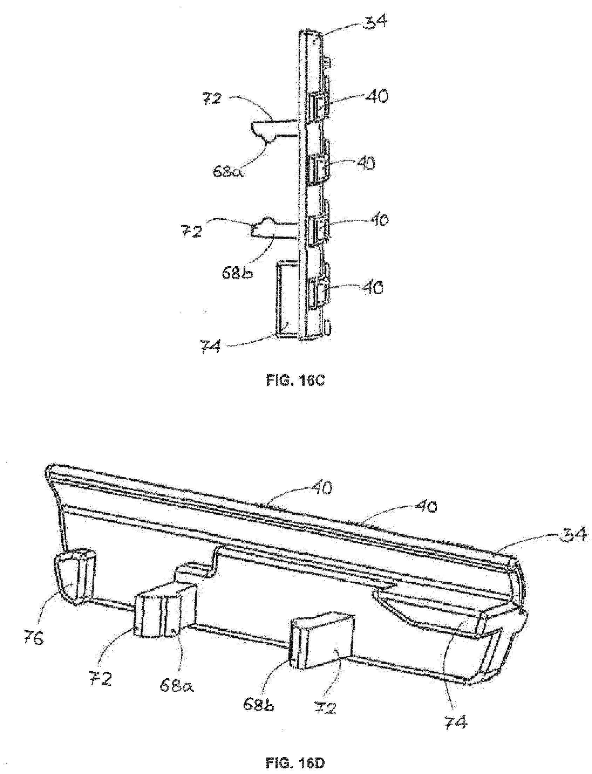

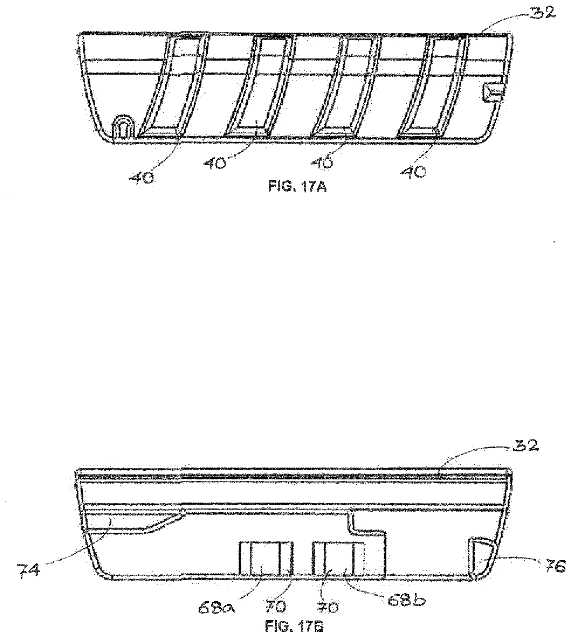

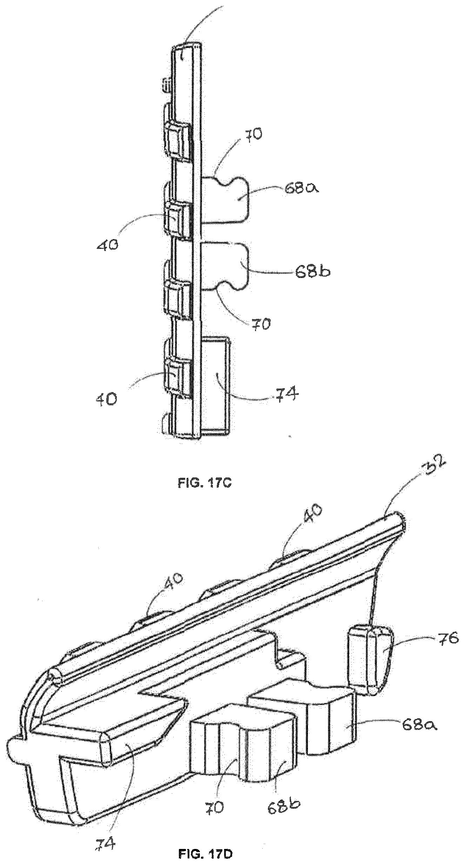

As can be seen in FIGS. 16 and 17 the first slider element 32 on its inner surface in the mid section 28 bottom part is provided with at least two projections 68a, 68b extending outwardly therefrom, the projections 68a, 68b each having lock formation 70 at its free end being capable of lockingly secure with the second 34 slider element when positioned in the recess 36 provided in the handle 12. Likewise the second slider 34 element on its inner surface in the mid-section 28 bottom part is provided with at least two projections 68a, 68b extending outwardly therefrom, the projections 68a, 68b each having catch formations 72 at its free end are adapted to receive the projections 68a, 68b of the first 32 slider element when positioned in the recess 36 provided in the handle 12. When secured in the recess 36 of the handle 12 the first and second 34 slider elements locks onto each other securing the blade carrier 26 within the bore 14 in the housing 16 and the blade carrier 26 can be slid forwards and rearwards by manually operating the first 32 and second 34 slider element holding the scalpel handle 12 with the use of fingers.

The inner surface of the slider elements 32, 34 is also provided with a forward tab 74 and rear tab 76. The forward tab 74 is positioned near the distal ends thereof and the rear tab 76 is positioned near the proximal end of the slider elements 32, 34. The forward tab 74 is adapted to engage the first 46 and second 48 flexible latching elements when the blade 30 is in-use position. The forward tab 74 prevents the blade carrier element 26 from moving the blade 30 forwardly beyond open position. Similarly, the rear tab 76 is adapted to engage the blade carrier element 26 from moving the blade 30 rearwardly beyond the closed position. Alternatively, the rear tab 76 prevents the blade carrier element 26 from moving the blade 30 rearwardly beyond the fully and/or permanently locked position. The first 32 and second 34 slider elements are received in the space defined by a laterally recessed 36 surface on the handle 12 forming a rim structure which extends about and forms the margin of said slot in a movably sliding arrangement. The outer surface of the first 32 and second 34 slider elements is also provided with finger grip formations in the forms of ribs 40, which facilitate a frictional contact with an individual's fingers facilitating grip support. The retractable universal safety scalpel 10 can be easily used / handled by both a right-handed as well as a left-handed person without having to alter the structure of the scalpel 10 and blade 30 due to their symmetrical configuration.

The retractable universal safety scalpel 10 constructed in accordance with present invention embodies the use of various sizes of blades 30 which may be permanently or releasably attached to the said blade carrier 26, depending on the nature of the use of the scalpel 10.

The handle 12 is preferably formed entirely of a plastic material and, in this form, includes a recess 36 extending in an axial direction - A in between the proximal 18a and distal 20a section of the handle 12.

By holding the handle 12 in the palm of the individual's hand and placing the fingers along the ribs 40 provided on the handle 12 and slider elements i.e., the first 32 and second 34 sliding elements may be advanced from pre-use position in the direction of distal 20a section to in-use position exposing the blade 30 out of the housing 16 of the handle 12 for use. The degree of control and movement of the slider elements 32, 34 corresponds with the degree of the blade 30 being exposed out of distal 20a section of the handle 12. In transitioning between the two positions, it will be appreciated that the handle 12 can be operated with only one hand, thus freeing the other hand for other work.

After use and when it is desirable to dispose of the scalpel 10, the slider elements 32, 34 may be advanced in the direction of proximal 18a section of the handle 12 into its permanently locked position. To accomplish this, the handle 12 is once again placed in the user's hand allowing the fingers to advance the sliding elements in the direction of proximal 18a section of the handle 12 to a permanently locked position. In the permanently locked position engaging means as provided in the blade 30 carrier element 26 and slider elements 32, 34 prompt to blocking rearward return movement of the sliding elements. Consequently, the sliding elements are permanently locked, covering the blade 30, whereby individuals are protected from inadvertent, casual and non-intentional contact with the blade 30.

Once the scalpel 10 is in permanently locked position, it is impossible to use the scalpel 10 again. The scalpel blade 30 is thus held in an extremely safe locked inoperative position for disposal, thereby avoiding the possibility of any so-called sharps injuries. The scalpel 10 may then be handled for further disposal without the danger attendant to an exposed blade 30.

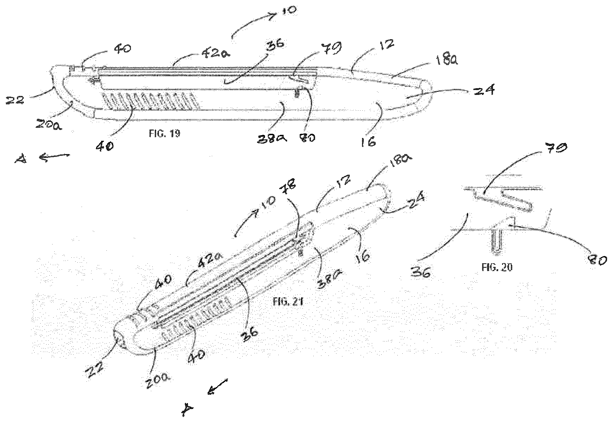

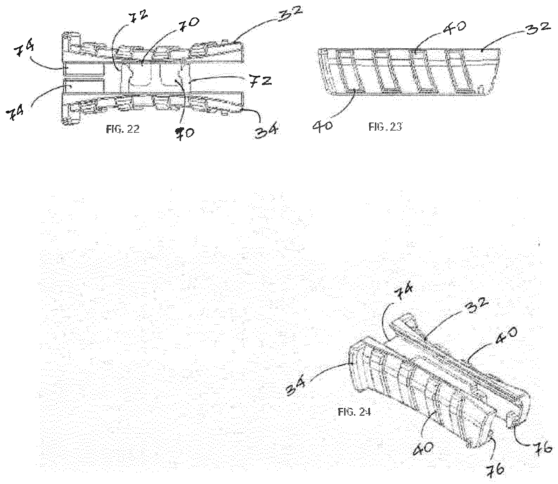

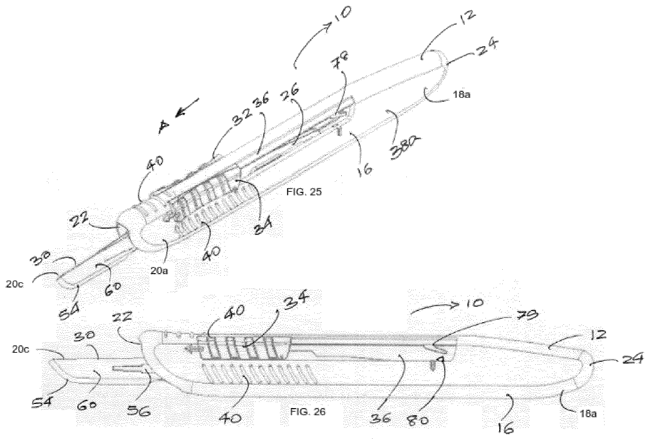

In one embodiment, as shown in Figs. 19 to 26, the housing 16 is provided with a bore 14 which holds the blade carrier element 26. The blade carrier element 26 is slideably arranged in the bore 14 from an un-exposed pre-use condition to an exposed in-use position and to an after-use locked disposal position where the blade is permanently locked. The safety scalpel 10 is provided with plurality of locking means to ensure locking of the blade carrier element in various positions in particular to ensure locking of the blade carrier element 26 in the housing in use position and to ensure permanent locking of the blade carrier element 26 with the blade 30 in a retracted position when the safety scalpel 10 is ready for disposal. As a result of the permanent disposal locking position, the safety of handlers during disposal is ensured. Similarly, the easy locking transfer between the in-use position to permanent locking position ensures that transfer of the safety scalpel 10 during operation does not result in any accidental nick or cut to the user or handler.

The locking means comprise at least a pair of locking members 78 formed integrally in the housing 16 provided in the housing, in particular in the region of the recess 36 proximal to the closed end 24 of the housing 16, being adapted to engagably receive the slider elements 32, 34 and retain them therein securely in a locked position. The said pair of locking members 78 shown in greater detail in Fig. 20 include at least one locking finger 79 and at least one locking tab 80 adapted to engagably receive the slider elements 32, 34 in particular the projections 68a, 68b and tabs 74, 76 provided thereon to ensure a permanent locking position. After final completion of the surgical procedure, the blade carrier element 26 is pushed back to a final permanent locking position with the help of slider elements 32, 34 where locking means including locking members 78 engage with the projections 68a, 68b and tabs 74, 76 provided on the slider elements 32, 34 to ensure permanent locking. In this final stop position, the blade carrier element 26 with the blade 30 cannot be released accidentally, thereby ensuring complete safety of the user.

The name "Paramount" appearing in the drawings is the trade mark of Paramount Surgimed Ltd.

Thus, the present invention provides for a retractable universal safety scalpel 10 having safety features which once activated cannot be deactivated and remains protective through disposal; perform reliably and easy to use and is thus safe and effective during surgical procedure and afterwards.

Although this invention has been disclosed in the context of certain preferred embodiments and examples, it will be apparent to one of ordinary skill in the art that many changes and modifications can be made thereto without departing from the scope of the invention as set forth in the appended claims.

Accordingly, it is not intended that the scope of the foregoing description be limited to the exact description set forth above, but rather should be determined only by a fair reading of the claims appended below.

## Claims

1. A retractable universal safety scalpel (10) comprising a handle (12) having a proximal section (18a) and a distal section (20a) with a longitudinally extending bore (14) defining a housing (16);
a blade carrier element (26) having mid-section (28), proximal end and a distal end on which is secured a blade (30) slidably mounted within said housing such that it can be exposed from the distal end section of said housing;
first and second slider elements (32, 34) secured to said blade carrier element through a recess (36) formed in the handle extending from a forward end closer to an opening (22) to a rear end closer to the distal section of the handle in a slidable arrangement with the housing and extending externally in an axial direction of said housing to move said blade carrier element moving the blade from an un-exposed pre-use position to an exposed in-use position and to an after-use position capable of covering and locking permanently the blade completely within the housing of the handle;
a plurality of locking means is provided in the housing to enable locking of the blade in an un-exposed pre-use position to an exposed in-use position and to an after-use position capable of covering and permanently locking the blade, wherein the housing is provided with lock cooperating mechanisms at three positions thereon corresponding to pre-use, in-use and post-use disposal stages, said lock cooperating mechanisms cooperating with the respective locking means provided thereon;
**characterised in that** the first and second slider elements (32, 34) comprise on their inner surfaces a forward tab (74) positioned near the distal end thereof and a rear tab (76) positioned near the proximal end thereof;
the blade carrier element (26) is provided with first and second flexible latching elements (46, 48) extending generally in angular opposing directions adjoining the mid-section (28) of the blade carrier element, such that when secured in the housing (16) the latching elements slidably engage with the first and second slider elements (32, 34); and
the housing comprises a pair of locking members (78) for engagably receiving the slider elements and retaining them securely in the permanently locked after-use position.

2. The retractable universal safety scalpel (10) as claimed in claim 1, wherein the housing (16) has an opening (22) at the distal end and a closed end at the proximal section (18a) of the handle (12).

3. The retractable universal safety scalpel (10) as claimed in claim 1 or claim 2, wherein the handle (12) at its sidewalls is provided with a recess (36) from the forward end closer to the opening (22) to the rear end closer to the distal section (20) of the handle.

4. The retractable universal safety scalpel (10) as claimed in any preceding claim, wherein the handle (12) is provided with finger grip formation on the top (42a) and side wall surfaces (38a, 38b) of the handle at the distal section (20a) thereof, said grip formation includes one or more ribs (40) forming frictional contact with an individual's fingers.

5. The retractable universal safety scalpel (10) as claimed in any preceding claim, wherein the blade (30) defines an elongated aperture near the distal end thereof.

6. The retractable universal safety scalpel (10) as claimed in claim 5, wherein the blade carrier element (26) comprises a mating protrusion (56) positioned near the distal end thereof such that the mating protrusion is adapted to engage the aperture of the blade (30).

7. The retractable universal safety scalpel (10) as claimed in any preceding claim, wherein the blade carrier element (26) comprises a front and back face having a mid-section (28) and distal end forming the forward part and proximal end forming the rear part, such that the front and back faces are dissimilar in profile.

8. The retractable universal safety scalpel (10) as claimed in claim 7, wherein the distal end forming the forward part and proximal end forming the rear part is widened compared with said mid-section (28) of the blade carrier element (26), such that top (42b) and bottom (44b) surfaces of the forward part slidably engage the top and bottom surfaces of the bore (14) within the housing (16).

9. The retractable universal safety scalpel (10) as claimed in claim 7 or claim 8, wherein the mid-section (28) has a longitudinally extending substantially bar shaped groove (64) on the front face, such that when the blade carrier element (26) is slidably arranged in the housing (16) the groove is received in a mating projection (66) formed on the inner side wall of the handle (12) and wherein the mid-section has a longitudinally extending substantially bar shaped projection on the back face, such that when the blade carrier element (26) is slidably arranged in the housing (16) the groove is received in a mating groove formed on the inner side wall of the handle.

10. The retractable universal safety scalpel (10) as claimed in claim 1, wherein the recess (36) has an increased cross-sectional area (50) having dimension of abutment (52), such that the first latching element (46) abuts against the abutment when the blade (30) is in its forward open position being exposed out of the opening (22).

11. The retractable universal safety scalpel (10) as claimed in any preceding claim, wherein the first slider element (32) on its inner surface in the mid-section (28) bottom part is provided with at least two projections (68a, 68b) extending outwardly therefrom, the projections each having lock formation (70) at its free end.

12. The retractable universal safety scalpel (10) as claimed in any preceding claim, wherein the second slider element (34) on its inner surface in the middle section bottom part is provided with at least two projections (68a, 68b) extending outwardly therefrom, the projections each having catch formations at their free ends, such that said catch formations are adapted to receive the projections of the first slider element (32) when positioned in the recess (36) provided in the handle (12).

13. The retractable universal safety scalpel (10) as claimed in any preceding claim, wherein the locking members are provided in the region of the recess (36) proximal to the closed end of the housing.

14. The retractable universal safety scalpel (10) as claimed in claim 1, wherein the forward tab (74) is adapted to engage the first and second flexible latching elements (46, 48) when the blade (30) is in an in-use position, and the rear tab (76) is adapted to engage the blade carrier element (26).

15. The retractable universal safety scalpel (10) as claimed in claim 14, wherein the rear tab (76) prevents the blade carrier element (26) from moving the blade (30) rearwardly beyond the after-use position.

## Patentansprüche

1. Einziehbares universelles Sicherheitsskalpell (10), Folgendes umfassend: einen Griff (12) mit einem proximalen Abschnitt (18a) und einem distalen Abschnitt (20a) mit einer sich längs erstreckenden Bohrung (14), die ein Gehäuse (16) definiert;
ein Klingenträgerelement (26) mit einem Mittelabschnitt (28), einem proximalen Ende und einem distalen Ende, an dem eine Klinge (30) befestigt ist, das derart verschiebbar in dem Gehäuse angebracht ist, dass es aus dem distalen Endabschnitt des Gehäuses herausgeschoben werden kann;
ein erstes und ein zweites Schieberelement (32, 34), die durch eine in dem Griff ausgebildete Aussparung (36) hindurch, die sich von einem vorderen Ende, das einer Öffnung (22) näher ist, bis zu einem hinteren Ende, das dem distalen Abschnitt des Griffs näher ist, erstreckt, in einer verschiebbaren Anordnung mit dem Gehäuse an dem Klingenträgerelement befestigt sind und sich außerhalb in einer axialen Richtung des Gehäuses erstrecken, um das Klingenträgerelement zu bewegen und dadurch die Klinge aus einer nicht herausgeschobenen Stellung vor ihrer Verwendung in eine herausgeschobene Verwendungsstellung und in eine Stellung nach ihrer Verwendung, die in der Lage ist, die Klinge vollständig in dem Gehäuse des Griffs zu verbergen und dauerhaft darin zu verriegeln, zu bewegen;
mehrere Verriegelungsmittel, die in dem Gehäuse bereitgestellt sind, um ein Verriegeln der Klinge in einer nicht herausgeschobenen Stellung vor ihrer Verwendung in eine herausgeschobene Verwendungsstellung und in eine Stellung nach ihrer Verwendung, die in der Lage ist, die Klinge zu verbergen und dauerhaft zu verriegeln, zu ermöglichen, wobei das Gehäuse an drei Positionen, die Anordnungsstufen vor, bei und nach Verwendung entsprechen, mit mit der Verriegelung zusammenwirkenden Mechanismen versehen ist, wobei die mit der Verriegelung zusammenwirkenden Mechanismen mit den daran bereitgestellten entsprechenden Verriegelungsmitteln zusammenwirken;
**dadurch gekennzeichnet, dass** das erste und das zweite Schieberelement (32, 34) auf ihren inneren Oberflächen einen vorderen Steg (74), der nahe dem distalen Ende davon positioniert ist, und einen hinteren Steg (76), der nahe dem proximalen Ende davon positioniert ist, umfassen;
das Klingenträgerelement (26) mit einem ersten und einem zweiten flexiblen Rastelement (46, 48) versehen ist, die sich, an den Mittelabschnitt (28) des Klingenträgerelements angrenzend, allgemein in winkligen entgegengesetzten Richtungen erstrecken, derart dass, wenn sie in dem Gehäuse (16) befestigt sind, die Rastelemente verschiebbar das erste und das zweite Schieberelement (32, 34) in Eingriff nehmen; und
das Gehäuse ein Paar Verriegelungselemente (78) zum Ineingriffnehmen der Schieberelemente und sicheren Festhalten derselben in der dauerhaft verriegelten Stellung nach Benutzung umfasst.

2. Einziehbares universelles Sicherheitsskalpell (10) nach Anspruch 1, wobei das Gehäuse (16) an dem distalen Ende eine Öffnung (22) aufweist und an dem proximalen Abschnitt (18a) des Griffs (12) ein geschlossenes Ende aufweist.

3. Einziehbares universelles Sicherheitsskalpell (10) nach Anspruch 1 oder 2, wobei der Griff (12) an seinen Seitenwänden von dem vorderen Ende, das der Öffnung (22) näher ist, bis zu dem hinteren Ende, das dem distalen Abschnitt (20) des Griffs näher ist, mit einer Aussparung (36) versehen ist.

4. Einziehbares universelles Sicherheitsskalpell (10) nach einem vorangehenden Anspruch, wobei der Griff (12) auf den oberen (42a) und seitlichen Wandoberflächen (38a, 38b) des Griffs an dem distalen Abschnitt (20a) desselben mit einem Fingergriffgebilde versehen ist, wobei das Griffgebilde eine oder mehrere Rippen (40) umfasst, die einen Reibungskontakt mit den Fingern einer Person ausbilden.

5. Einziehbares universelles Sicherheitsskalpell (10) nach einem vorangehenden Anspruch, wobei die Klinge (30) nahe ihrem distalen Ende eine lang gestreckte Öffnung definiert.

6. Einziehbares universelles Sicherheitsskalpell (10) nach Anspruch 5, wobei das Klingenträgerelement (26) einen passenden Vorsprung (56) umfasst, der derart nahe dem distalen Ende desselben positioniert ist, dass der passende Vorsprung dazu ausgelegt ist, in die Öffnung der Klinge (30) einzugreifen.

7. Einziehbares universelles Sicherheitsskalpell (10) nach einem vorangehenden Anspruch, wobei das Klingenträgerelement (26) eine Vorder- und eine Rückseite mit einem Mittelabschnitt (28) und einem distalen Ende, das den vorderen Teil ausbildet, und einem proximalen Ende, das den hinteren Teil ausbildet, umfasst, derart dass die Vorder- und Rückseite in ihrem Profil unterschiedlich sind.

8. Einziehbares universelles Sicherheitsskalpell (10) nach Anspruch 7, wobei das distale Ende, das den vorderen Teil ausbildet, und das proximale Ende, das den hinteren Teil ausbildet, im Vergleich zu dem Mittelabschnitt (28) des Klingenträgerelements (26) derart verbreitert sind, dass eine obere (42b) und eine untere (44b) Oberfläche des vorderen Teils verschiebbar die obere und die untere Oberfläche der Bohrung (14) in dem Gehäuse (16) in Eingriff nehmen.

9. Einziehbares universelles Sicherheitsskalpell (10) nach Anspruch 7 oder 8, wobei der Mittelabschnitt (28) auf der Vorderseite eine sich längs erstreckende im Wesentlichen streifenförmige Nut (64) aufweist, derart dass, wenn das Klingenträgerelement (26) verschiebbar in dem Gehäuse (16) angeordnet ist, die Nut in einem passenden Vorsprung (66) aufgenommen wird, der auf der inneren Seitenwand des Griffs (12) ausgebildet ist, und wobei der Mittelabschnitt auf der Rückseite einen sich längs erstreckenden im Wesentlichen streifenförmigen Vorsprung aufweist, derart dass, wenn das Klingenträgerelement (26) verschiebbar in dem Gehäuse (16) angeordnet ist, die Nut in einer passenden Nut aufgenommen wird, die auf der inneren Seitenwand des Griffs ausgebildet ist.

10. Einziehbares universelles Sicherheitsskalpell (10) nach Anspruch 1, wobei die Aussparung (36) eine vergrößerte Querschnittsfläche (50) aufweist, die eine Abmessung eines Anschlags (52) aufweist, derart dass das erste Rastelement (46) an dem Anschlag anliegt, wenn die Klinge (30) in ihrer vorderen offenen Stellung ist, in der sie aus der Öffnung (22) herausgeschoben ist.

11. Einziehbares universelles Sicherheitsskalpell (10) nach einem vorangehenden Anspruch, wobei das erste Schieberelement (32) auf seiner inneren Oberfläche im unteren Teil des Mittelabschnitts (28) mit wenigstens zwei Vorsprüngen (68a, 68b) versehen ist, die sich davon nach außen erstrecken, wobei die Vorsprünge an ihrem freien Ende jeweils ein Verriegelungsgebilde (70) aufweisen.

12. Einziehbares universelles Sicherheitsskalpell (10) nach einem vorangehenden Anspruch, wobei das zweite Schieberelement (34) auf seiner inneren Oberfläche im unteren Teil des Mittelabschnitts mit wenigstens zwei Vorsprüngen (68a, 68b) versehen ist, die sich davon nach außen erstrecken, wobei die Vorsprünge an ihren freien Enden jeweils Haltegebilde aufweisen, derart dass die Haltegebilde dazu ausgelegt sind, die Vorsprünge des ersten Schieberelements (32) aufzunehmen, wenn sie in der in dem Griff (12) bereitgestellten Aussparung (36) positioniert sind.

13. Einziehbares universelles Sicherheitsskalpell (10) nach einem vorangehenden Anspruch, wobei die Verriegelungselemente im Bereich der Aussparung (36) nahe dem geschlossenen Ende des Gehäuses bereitgestellt sind.

14. Einziehbares universelles Sicherheitsskalpell (10) nach Anspruch 1, wobei der vordere Steg (74) dazu ausgelegt ist, das erste und das zweite flexible Rastelement (46, 48) in Eingriff zu nehmen, wenn die Klinge (30) in einer Verwendungsstellung ist, und der hintere Steg (76) dazu ausgelegt ist, das Klingenträgerelement (26) in Eingriff zu nehmen.

15. Einziehbares universelles Sicherheitsskalpell (10) nach Anspruch 14, wobei der hintere Steg (76) verhindert, dass das Klingenträgerelement (26) die Klinge (30) über die Stellung nach Verwendung hinaus nach hinten bewegt.

## Revendications

1. Scalpel de sécurité universel rétractable (10) comprenant un manche (12) ayant une section proximale (18a) et une section distale (20a) avec un alésage s'étendant longitudinalement (14) définissant un logement (16) ;
un élément porte-lame (26) ayant une section milieu (28), une extrémité proximale et une extrémité distale sur lesquelles est assujettie une lame (30) montée en coulissement au sein dudit logement de telle sorte qu'elle puisse être exposée depuis ladite section d'extrémité distale dudit logement ;
des premier et second éléments de coulisse (32, 34) assujettis audit élément porte-lame par l'intermédiaire d'un évidement (36) formé dans le manche s'étendant d'une extrémité avant plus proche d'une ouverture (22) à une extrémité arrière plus proche de la section distale du manche dans un agencement coulissant avec le logement et s'étendant à l'extérieur dans une direction axiale dudit logement pour déplacer ledit élément porte-lame déplaçant la lame d'une position préutilisation non exposée à une position en cours d'utilisation exposée et à une position post-utilisation capable de couvrir et de bloquer en permanence la lame complètement au sein du logement du manche ;
une pluralité de moyens de blocage est ménagée dans le logement pour permettre un blocage de la lame dans une position préutilisation non exposée à une position en cours d'utilisation exposée et à une position post-utilisation capable de couvrir et de bloquer en permanence la lame, le logement étant pourvu de mécanismes coopérant en blocage en trois positions correspondant à des étages de disposition préutilisation, en cours d'utilisation et post-utilisation, lesdits mécanismes coopérant en blocage coopérant avec les moyens de blocage respectifs ménagés dessus ;
**caractérisé en ce que** les premier et second éléments de coulisse (32, 34) comprennent sur leurs surfaces internes une patte avant (74) positionnée près de leur extrémité distale et une patte arrière (76) positionnée près de leur extrémité proximale ;
l'élément porte-lame (26) est pourvu de premier et second éléments de verrouillage flexibles (46, 48) s'étendant généralement dans des directions opposées angulaires attenantes à la section milieu (28) de l'élément porte-lame, de telle sorte qu'une fois assujettis dans le logement (16), les éléments de verrouillage se mettent en prise en coulissement avec les premier et second éléments de coulisse (32, 34) ; et
le logement comprend une paire d'organes de blocage (78) permettant de recevoir en prise les éléments de coulisse et de les retenir solidement dans la position post-utilisation bloquée en permanence.

2. Scalpel de sécurité universel rétractable (10) selon la revendication 1, dans lequel le logement (16) comporte une ouverture (22) à l'extrémité distale et une extrémité fermée au niveau de la section proximale (18a) du manche (12).

3. Scalpel de sécurité universel rétractable (10) selon la revendication 1 ou la revendication 2, dans lequel le manche (12) au niveau de ses parois latérales est pourvu d'un évidement (36) de l'extrémité avant plus proche de l'ouverture (22) à l'extrémité arrière plus proche de la section distale (20) du manche.

4. Scalpel de sécurité universel rétractable (10) selon l'une quelconque des revendications précédentes, dans lequel le manche (12) est pourvu d'une formation de poignée sur les surfaces de paroi haute (42a) et latérale (38a, 38b) du manche à leur section distale (20a), ladite formation de poignée inclut une ou plusieurs nervures (40) formant un contact par frottement avec les doigts d'un individu.

5. Scalpel de sécurité universel rétractable (10) selon l'une quelconque des revendications précédentes, dans lequel la lame (30) définit une ouverture allongée près de son extrémité distale.

6. Scalpel de sécurité universel rétractable (10) selon la revendication 5, dans lequel l'élément porte-lame (26) comprend une protubérance d'accouplement (56) positionnée près de son extrémité distale de telle sorte que la protubérance d'accouplement soit adaptée pour mettre en prise l'ouverture de la lame (30).

7. Scalpel de sécurité universel rétractable (10) selon l'une quelconque des revendications précédentes, dans lequel l'élément porte-lame (26) comprend une face frontale et une face de dos ayant une section milieu (28) et une extrémité distale formant la partie avant et l'extrémité proximale formant la partie arrière, de telle sorte que les faces frontales et de dos soient de profil dissemblable.

8. Scalpel de sécurité universel rétractable (10) selon la revendication 7, dans lequel l'extrémité distale formant la partie avant et l'extrémité proximale formant la partie arrière est élargie en comparaison à ladite section milieu (28) de l'élément porte-lame (26), de telle sorte que des surfaces haute (42b) et basse (44b) de la partie avant mettent en prise en coulissement les surfaces haute et basse de l'alésage (14) au sein du logement (16).

9. Scalpel de sécurité universel rétractable (10) selon la revendication 7 ou la revendication 8, dans lequel la section milieu (28) comporte une rainure en forme de barre s'étendant sensiblement longitudinalement (64) sur la face frontale, de telle sorte que lorsque l'élément porte-lame (26) est agencé en coulissement dans le logement (16), la rainure est reçue dans une saillie d'accouplement (66) formée sur la paroi latérale interne du manche (12) et dans lequel la section milieu comporte une saillie en forme de barre s'étendant sensiblement longitudinalement sur la face de dos, de telle sorte que lorsque l'élément porte-lame (26) est agencé en coulissement dans le logement (16), la rainure est reçue dans une rainure d'accouplement formée dans la paroi latérale interne du manche.

10. Scalpel de sécurité universel rétractable (10) selon la revendication 1, dans lequel l'évidement (36) a une aire en coupe accrue (50) ayant la dimension d'une butée (52), de telle sorte que le premier élément de verrouillage (46) vienne en butée contre la butée lorsque la lame (30) est dans sa position ouverte avant qui est exposée hors de l'ouverture (22).

11. Scalpel de sécurité universel rétractable (10) selon l'une quelconque des revendications précédentes, dans lequel le premier élément de coulisse (32) sur sa surface interne dans la partie basse de la section milieu (28) est pourvu d'au moins deux saillies (68a, 68b) sortant de celle-ci, les saillies comportant chacune une formation de blocage (70) à son extrémité libre.

12. Scalpel de sécurité universel rétractable (10) selon l'une quelconque des revendications précédentes, dans lequel le second élément de coulisse (34) sur sa surface interne dans la partie basse de la section milieu est pourvu d'au moins deux saillies (68a, 68b) sortant de celle-ci, les saillies ayant chacune des formations d'accroche à leurs extrémités libres, de telle sorte que lesdites formations d'accroche sont adaptées pour recevoir les saillies du premier élément de coulisse (32) lorsqu'il est positionné dans l'évidement (36) ménagé dans le manche (12).

13. Scalpel de sécurité universel rétractable (10) selon l'une quelconque des revendications précédentes, dans lequel les organes de blocage sont ménagés dans la région de l'évidement (36) à proximité de l'extrémité fermée du logement.

14. Scalpel de sécurité universel rétractable (10) selon la revendication 1, dans lequel la patte avant (74) est adaptée pour mettre en prise les premier et second éléments de verrouillage flexibles (46, 48) lorsque la lame (30) est dans une position en cours d'utilisation, et la patte arrière (76) est adaptée pour mettre en prise l'élément porte-lame (26).

15. Scalpel de sécurité universel rétractable (10) selon la revendication 14, dans lequel la patte arrière (76) empêche l'élément porte-lame (26) de déplacer la lame (30) vers l'arrière au-delà de la position post-utilisation.
